# EUROPEAN PATENT APPLICATION

(11) **EP 2 533 045 A1**
(43) Date of publication of application: **12.12.2012**
(21) Application number: 11305708.7
(22) Date of filing: 08.06.2011
(51) Int. Cl.: G01N 33/50, G01N 33/569, C07K 14/47

(54) **Methods for identifying adult stem cells**

(71) Applicant: Université Pierre et Marie Curie Paris 6, 75005 Paris (FR)
(72) Inventor: Sassoon, David, 75007 Paris (FR); Marazzi, Giovanna, 75007 Paris (FR); Besson, Vanessa, 78160 Marly le Roi (FR)
(74) Representative: Chajmowicz, Marion

(57) **Abstract**

The present invention relates to the use of anti-PW1 antibodies for identifying adult stem cells, screening pharmaceutical drugs that act upon the stem cells, and monitoring cell aging.

## Description

The present invention relates to methods for identifying adult stem cells, screening pharmaceutical drugs that act upon the stem cells, and monitoring stem cell competence in physiological and pathological conditions, including aging.

### Background of the invention

Stem cells are defined by the ability to continuously self-renew and produce the differentiated progeny of the tissue of their location (Morrison et al., 1997). Adult stem cells are undifferentiated cells that reside in differentiated tissues, and have the properties of self-renewal and generation of differentiated cell types. The differentiated cell types may include all or some of the specialized cells in the tissue. Sources of somatic stem cells include bone marrow, blood, the cornea and the retina of the eye, brain, skeletal muscle, dental pulp, liver, skin, the lining of the gastrointestinal tract, and pancreas. Adult stem cells are a small percentage of the total cells. For instance, in the small intestine there are perhaps up to 10 stem cells at the bottom of the crypt out of a total crypt population of <300 cells. In skeletal muscle, satellite (stem) cells comprise about 5% of all nuclei, but in the bone marrow the multi-potential hematopoietic stem cell is much rarer, with a frequency of perhaps 1 in 10000 amongst all bone marrow cells. Considerable overlap exists between different putative organ specific stem cells in their repertoire of gene expression, often related to self-renewal, cell survival, and cell adhesion. More robust tests of 'stemness' are now being employed, using lineage-specific genetic marking and tracking to show production of long-lived clones *in vitro* and multi-potentiality *in vivo.* However, the conditions to grow or simply 'select' stem cells *in vitro* do not exist for many tissues where it is accepted that the stem cells simply fail to grow due to lack of required growth factors or substrates. In many tissues, the stem cells have simply not been identified.

Nevertheless stem cells have been used routinely for more than three decades to repair tissues and organs damaged by injury or disease, most notably from bone marrow. While early, embryonic stem cells have generated considerable interest, adult stem cells are critical for tissue homeostasis and wound repair and reside within specific niches that preserve proliferative and regenerative potential (Blanpain and Fuchs, 2006; Moore and Lemischka, 2006).

Understanding how stem cells are maintained, stimulated and participate in regeneration is important to a wide variety of diseases as well as potential targets in the aging population.

However, very few markers of adult stem cells have been identified so far. For instance colon cancer research has led to the identification of leucine-rich repeat-containing G-protein coupled receptors Lgr5 and Lgr6 that are expressed by adult stem cells ((Barker et al., 2010); international patent application WO2009/022907). A population of muscle-resident stem cells was identified in the interstitium that expresses the cell stress mediator PW1/PEG3 but not other markers of muscle stem cells such as Pax7. PW1(+)/Pax7(-) interstitial cells (PICs) are myogenic *in vitro* and efficiently contribute to skeletal muscle regeneration *in vivo* as well as generating satellite cells and PICs. Furthermore, it was found that PICs are not derived from a satellite cell lineage. Taken together, these findings uncover an anatomically identifiable population of muscle progenitors (Mitchell et al., 2010). To characterize the role of *Pw1* as a potential marker of multiple stem cell populations, a reporter mouse, called *Tg(Pw1^{IRES-nLacZ}),* was generated (Besson et al, abstract published in April 2008, New-Orleans (New directions in Biology and Disease of skeletal muscle).

There remains a need for methods for identifying and culturing adult stem cells, and for rapid and reliable identification or screening of substances that act on adult stem cells.

### Summary of the invention

The present invention relates to the use of anti-PW1 antibodies for identifying adult stem cells, screening pharmaceutical drugs that act upon the stem cells, and monitoring stem cell competence.

The invention provides an *in vitro* method for screening a candidate substance for its ability to stimulate adult stem cells, which method comprises the steps consisting of:
a. Providing a tissue or adult stem cells;
b. Contacting said tissue or cells with the candidate substance;
c. Comparing a binding signal of an anti-PW1 antibody that is detectably labeled, before and after the contacting of said tissue or cell culture with the candidate substance; wherein the ability of the candidate substance to trigger, maintain or enhance the binding signal identifies the substance as being able to stimulate adult stem cells or to maintain competence of adult stem cells.

The invention further provides a method for screening a candidate substance for its ability to stimulate adult stem cells, which method comprises the steps consisting of:
a. providing a tissue section or biopsy of a subject who has been administered with the candidate substance;
b. contacting said tissue section or biopsy with an anti-PW1 antibody; wherein the ability of the candidate substance to trigger, maintain or enhance the binding signal of the anti-PW1 antibody that is detectably labeled identifies the substance as being able to stimulate adult stem cells or to maintain competence of adult stem cells.

It is further provided a method for identifying adult stem cells in a subject, which method comprises
a. obtaining a tissue section or biopsy of a subject;
b. contacting the tissue section of biopsy with an anti-PW1 antibody ;
c. visualizing the anti-PW1 antibody, whereby adult stem cells are identified.

The invention makes use of an anti-PW1 antibody for screening a candidate substance for its ability to stimulate adult stem cells.

The invention further makes use of an anti-PW1 antibody for monitoring stem cell competence in physiological and pathological conditions, including aging.

### Legends to the Figures

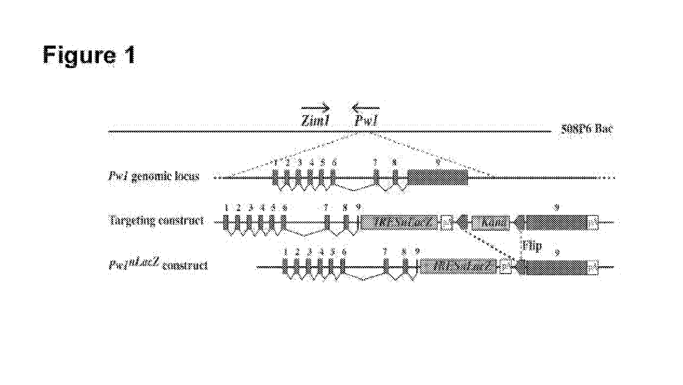

**Figure 1** is a schematic drawing of the BAC recombination strategy.
   Reporter mice have been generated using BAC recombineering strategy by introducing an IRESnLacZ cassette in the 5'part of the BAC exon 9. The BAC has been randomly integrated in the genome by classical transgenesis methods known to those in the field. The wild-type *Pw1* locus with its open reading frame from exon 1 to 9 (black boxes) is located into the 508P6 BAC (180 kb). *Zim1* gene is located at less than 40 kb from *Pw1.* The arrows indicate the direction of gene transcription. An *IRESnLacZ-pA* cassette and a *kanamycin* (*Kana*) gene floxed by two FRT sites (triangles) were introduced into the 5' part of *Pw1* exon 9. The *Tg(Pw1^{IRES-nLacZ})* BAC construct was generated by FRT sites recombination and injected in mice to establish the reporter line.
Figure 2 shows photomicrographs of representative mouse tissues cross-sections immunostained for PW1, showing the expression of PW1 in tissue specific stem cells. Muscle cross-sections immunostained for ß-galactosidase (red), Pw1 (green) and laminin (orange). Nuclei were counterstained with DAPI (blue). PW1 reporter co-localizes perfectly with PW1 protein.
**Figure 3A** shows a Western blot using lysate of competent or non-competent mouse mesoangioblast clones cultured in vitro. PW1 protein is present only in competent clones. β-tubulin antibody is used as a positive control.
Figure 3B shows Western blot using lysate of competent or non-competent human mesoangioblast clones cultured in vitro. PW1 protein is present only in competent clone. β-tubulin antibody is used as a positive control.

### Detailed description of the invention

### Anti-PW1 antibodies

*Pw1*/*Peg3* ("paternally expressed gene 3"), herein designated as *"Pw1",* is a maternally imprinted gene that is expressed primarily during embryogenesis and in adult ovary, testis, muscle, and brain in mouse. In the present invention, the term *"Pw1" or "Peg3* " means the mouse *Pw1* gene or the orthologous gene in any other animal species, in particular in humans.

Mammalian imprinting regulates growth and the establishment of parental nurturing behaviors, but the detailed molecular mechanisms by which this occurs are incompletely known. *Pw1* mediates cell stress and pro-survival pathways *in vitro,* as well as muscle atrophy and stem cell number *in vivo.* Kim et al. (2000) mapped the mouse *Pw1*/*Peg3* gene to proximal chromosome 7 and determined that the gene contains 13 exons, the last 4 of which originated from the ancestral ZIM2 gene (Kim et al., 2000). The initiation codon is located in exon 3. Because imprinting is generally conserved among mammals, and imprinted domains generally encompass several adjacent genes, expression patterns and chromosomal environment of the human counterpart of *Peg3* was of interest. Kim et al. (1997) localized the human *PW1*/*PEG3* gene approximately 2 Mb proximal to the telomere of 19q, within a region known to carry large numbers of tandemly clustered Kruppel-type zinc finger-containing (ZNF) genes (Kim et al., 1997).

The protein encoded by the Pw1 gene is named the "PW1 protein". This protein has been conserved during evolution. PW1 protein sequence identity is estimated at 63.9% between human and mouse. In the present invention, the term "PW1" means the mouse Pw1 protein or the orthologous gene in any other animal species, in particular in humans.

Mouse PW1 protein sequence is shown as SEQ ID NO:1, and human PW1 protein sequence is shown as SEQ ID NO:2.

The N-terminus part of human PW1 protein is sufficiently different to allow producing antibodies that specifically bind the human PW1 protein. SEQ ID NO: 3 shows a N-term aminoacid sequence that is useful for that purpose.

Antibodies against the PW1 protein can be produced using the standard protocols known in the art. Antibodies may be monoclonal or polyclonal antibodies.

In a preferred embodiment, the anti-PW1 antibody is prepared as described in Relaix et al, 1996.

Generally speaking, monoclonal antibodies may be prepared from various mammalian hosts such as mice, rodents, primates, humans, etc, as well as birds, e.g. chicken. Description of techniques for preparing such monoclonal antibodies may be found in, e.g., Stites, et al. (eds.) Basic and Clinical Immunology (4th ed.), Lange Medical Publications, Los Altos, Calif., and references cited therein; Harlow and Lane (1988) Antibodies: A Laboratory Manual, CSH Press; Goding (1986) Monoclonal Antibodies: Principles and Practice (2d ed.) Academic Press, New York; and particularly in Kohler and Milstein (1975) in Nature 256:495-497, which discusses one method of generating monoclonal antibodies. Summarized briefly, this method involves injecting an animal with an immunogen. The animal is then sacrificed and cells taken from its spleen, which are then fused with myeloma cells. The result is a hybrid cell or "hybridoma" that is capable of reproducing in vitro. The population of hybridomas is then screened to isolate individual clones, each of which secrete a single antibody species to the immunogen. In this manner, the individual antibody species obtained are the products of immortalized and cloned single B cells from the immune animal generated in response to a specific site recognized on the immunogenic substance.

Other suitable techniques involve in vitro exposure of lymphocytes to the antigenic polypeptides or alternatively to selection of libraries of antibodies in phage or similar vectors. See, Huse, et al. (1989) "Generation of a Large Combinatorial Library of the Immunoglobulin Repertoire in Phage Lambda," Science 246:1275-1281; and Ward, et al. (1989) Nature 341:544-546. The antibodies of the present invention may be used with or without modification, including chimeric or humanized antibodies.

The antibodies according to the present invention are preferably specific monoclonal or polyclonal antibodies which can be obtained according to the standard methods well known to the person skilled in the art.

In particular embodiments, one makes use of antibody fragments, such as Fv, scFv (sc for single chain), Fab, F(ab')₂, Fab', scFv-Fc fragments or diabodies.

The anti-PW1 antibody may be detectably labeled.

As used herein, the term "labeled" or "detectably labeled", with regard to an antibody, is intended to encompass direct labeling by coupling (i.e., physically linking) a detectable substance, such as a radioactive agent or a chromophore (e.g. alkaline phosphatase or peroxidase such as HRP) or a fluorophore (e.g. fluorescein isothiocyanate (FITC) or phycoerythrin (PE) or Indocyanine (Cy5)) to the ligand, as well as indirect labeling by reactivity with a detectable substance or with an anti-IgG antibody that is itself detectably labeled with a detectable substance.

The term "binding signal" refers to the signal emitted by the detectable substance that identifies the anti-PW1 antibody. Washing the free anti-PW1 antibody may be needed when the bound anti-PW1 antibody only is to be detected.

### Identification of adult stem cells and screening methods

The anti-PW1 antibodies used in the present invention provides setting in which stem cells can be immediately recognized in their normal tissue context or in response to experimental manipulation. These antibodies can be used to identify the quiescent and/or proliferative stem cells and their niche in all adult tissues.

In a first embodiment, the invention provides a method for identifying adult stem cells in a subject, which method comprises
a. obtaining a tissue section or biopsy of a subject;
b. contacting the tissue section of biopsy with anti-PW1 antibody;
c. visualizing the anti-PW1 antibody, whereby adult stem cells are identified.

The tissue section or biopsy is obtained from a tissue selected from the group consisting of blood, bone marrow, hematopoietic system, skin, hair follicle, muscle, nervous system, heart, intestine, thymus, pancreas, testis, eye, kidney, liver, lung, spleen, tongue, bones, dental pulp, breast, ovaries, uterus, and placenta.

The anti-PW1 antibodies are useful for screening or identifying a candidate substance for its ability to stimulate adult stem cells.

The candidate substance may be any substance of defined or undefined structure, including a chemical drug, a biological compound, e.g. antibodies, nucleic acids or, peptides, or a mixture of natural compounds, e.g. an extract of a plant. In a preferred embodiment, it is a pharmaceutical drug, *i*.*e*. a drug that is pharmaceutically acceptable.

The candidate substance that is hereby selected is of interest in tissue repair, which may be useful in treating neurodegenerative diseases, including stroke and Alzheimer's disease, in spinal cord injury, as well as cardiovascular diseases, in particular myocardial infarction. Another field of regenerative medicine is skin repair, in particular for bums or genetic diseases.

The screening of candidate substances may be performed *in vitro* or *in vivo.* According to the invention, candidate substances are evaluated for their ability to trigger or maintain the binding signal.

More particularly, the invention provides an *in vitro* method for screening a candidate substance for its ability to stimulate adult stem cells, which method comprises the steps consisting of:
a. Providing a tissue or adult stem cells;
b. Contacting said tissue or cells with the candidate substance;
c. Comparing a binding signal of an anti-PW1 antibody that is detectably labeled, before and after the contacting of said tissue or cell culture with the candidate substance; wherein the ability of the candidate substance to trigger, maintain or enhance the binding signal identifies the substance as being able to stimulate or to maintain competence of adult stem cells.

The term "competence of adult stem cells" refers to their properties of self-renewal and generation of differentiated cell types.

The screening methods of the invention may be performed on tissues or on adult stem cells. In the context of the present invention, the term "adult stem cells" include induced pluripotent stem cells, which are artificially derived from a non-pluripotent cell, typically an adult somatic cell.

The tissue may be obtained from any human or non-human animal, preferably a mammal, including rodent, sheep, goats, cattle, horses, dogs, cats, primates.

The tissue may be of any type and origin. The tissue or stem cells of step (a) may be obtained or isolated from any tissue such as blood, bone marrow, hematopoietic system, skin, hair follicle, muscle, nervous system, heart, intestine, thymus, pancreas, testis, eye, kidney, liver, lung, spleen, tongue, bones, dental pulp, breast, ovaries, uterus, and placenta. Preferably, the tissue is in the form of a section or a biopsy.

Adult stem cells may have been previously isolated, and optionally purified, using surface markers such as P CD34, alpha integrin, cKit, Lin, Sca, etc.

More particularly the stem cells of step (a) may be isolated from an animal that has been subjected to a stress, consisting preferably of hypoxia, NO-induced stress, H2O2-induced stress, thermal stress *(e.g.* at about 42°C), or a chemically induced stress, e.g. with a histone inhibitor. Such stress impacts cell stem function and PW1 expression.

Protein preparation of the isolated tissues or isolated stem cells may be performed. Stem cells competence and/or mobilization is identified by the anti-PW1 antibody.
The binding of the anti-PW1 antibody may be determined by any standard method, including Western Blot or immunoprecipitation.

The bound antibody may be visualized either directly or indirectly, e.g. using a labeled anti-IgG antibody, by any standard method.

When the screening method is performed on tissues (such as sections or biopsies) stained with the anti-PW1 antibody, visualization of the binding may be achieved by standard methods, including immunohistochemistry, or immunofluorescence.
Again the bound antibody may be visualized either directly or indirectly, e.g. using a labeled anti-IgG antibody, by any standard method.
In a particular embodiment, the anti-PW1 antibody may be labeled with chromophore or fluorescent molecules and microscopy imaging methods are used to reveal the labeling.

The invention further provides an *in vivo* method for screening a candidate substance for its ability to stimulate adult stem cells, which method comprises administering a human or no-human animal subject, with the candidate substance; and analyzing the impact of the substance on the binding signal of an anti-PW1 antibody.
The ability of the candidate substance to trigger, maintain or enhance the binding signal identifies the substance as being able to stimulate adult stem cells.
Biopsies or sections, or whole mount staining of the subject with the antibody are performed before and/or after administering the candidate substance. Binding of the antibody to adult stem cells is then revealed.

Before, during, or sequentially with, administration of the candidate substance, the subject, preferably a non-human animal or a human patient, may be subjected to a stress, e.g. an environmental or genetic stress which impacts cell stem function, and PW1 expression. Preferably the patient suffers from a degenerative disease.

### Marker of cell aging

A further subject of the invention is the use of an anti-PW1 antibody for monitoring cell aging. Using anti-PW1 antibodies indeed allows to follow changes in expression of Pw1 as a function of age.
Especially the antibodies are useful tools for monitoring aging of adult stem cells present in skin or hair follicle.
The term "monitoring cell aging" includes studying changes in stem cell function as a model for human aging, in particular human skin aging.

The Examples and Figures illustrate the invention without limiting its scope.

### Examples

### Example 1: PW1/Peg3 identifies multiple adult stem and progenitor cell populations

The inventors have generated a transgenic reporter mouse model using BAC recombining strategy, as previously described (Lee et al., 2001). The Pw1-containing BAC clone (ID# 508P6, 180 kb) comes from 129Sv library. The BAC contains the 26,5 kb corresponding to Pw1 gene and the BAC contains at least 80 kb of sequence in the 5' and 3' part of Pw1. A kanamycine cassette surrounded by FRT sites was introduced into an IRESnLacZpA containing plasmid (3,8 kb) (Relaix 2004). The resulting cassette was subcloned into the XbaI site of pBSK plasmid containing a 485 bp homologous sequence (5' of exon 9 of genomic pw1, location: + 19302 bp of Pw1 genomic sequence, accession no. ENSMUSG00000002265; NCBIM37). The cassette was introduced in bacterial cells containing the BAC by electroporation. Selected colonies (Kanamycin positive cells) containing the targeting construct were submitted to arabinose, leading to the excision of the kanamycin cassette. Finally, the Tg(Pw1IRES-nLacZ) BAC was injected into ovocytes to generate founders. Germline-transmitted allele was identified by PCR (primer_Ex9: 5'-CCACATTCCTTACACTCTAAAGC-3' (SEQ ID NO:4) and primer_dLacZ: 5'-CCGCTACAGTCAACAGCAAC-3' (SEQ ID NO:5)). The Tg(Pw1IRES-nLacZ) reporter mice are maintained in a C57BL6/J background.

This mouse model identified quiescent or proliferative stem cell niches in adult tissues examined: muscle, cells from the hematopoietic system, intestine, hair follicle, central nervous system, epicardium, bone and, testis.

Using Tg(Pw1IRES-nLacZ/+) mice, the inventors have isolated skin stem cells to 98% purity using FACs analyses coupled with a fluorescent substrate for ß-galactosidase. The purified cells are long-lived and can be maintained in culture for several passages. Clonogenicity assays in vitro and graft experiments in vivo show that PW1 participates in hair follicle regeneration and the PW1+ cells display all the hallmarks of hair follicle stem cells. These results indicate that these purified blue stem cells can be cultured in vitro while keeping their stemness and thus their capacity of regeneration in vivo.

### Example 2: PW1 antibody identifies stem cells in several tissues

### 2.1. Material and methods

### Production of antibodies

The C-terminus portion of mouse Pw1 cDNA (residues 1180-1379) was subcloned into the pGEX expression vector (Pharmacia). Overnight cultures of the recombinant pGEX plasmid were diluted 1/10 and grown for 2 hr at 377C. IPTG (0.1 mM) was added and cells were allowed to grow an additional 4 hr. Cells were pelletted and lysates were prepared and absorbed onto glutathione agarose beads (Pharmacia). The glutathione transferase―Pw1 (GST-Pw1) fusion protein was eluted according to manufacturer specifications. The eluted protein was purified on a 7.5% acrylamide gel. Polyclonal antibodies were raised in rabbits by subcutaneous injection of 500 mg fusion protein (HRP, Inc. antisera system). Subsequent boosts were performed at 4-week intervals with 125 mg of protein. Antibodies against the fusion protein were obtained in two different rabbits (HM370 and HM371). Both antisera were purified on DEAE AffiGel (Bio-Rad) following manufacturer specifications.

### Immunohistrochemistry/Immunofluorescence

Tissues were fixed with 4% paraformaldehyde (PFA, w/v) in PBS at 4°C and placed in 15% PBS-sucrose overnight at 4°C before being embedded in OCT, except for the tibialis anterior muscles which were snap frozen in liquid nitrogen-cooled isopentane without previous fixation. Cryosections (8 µm) and cytospin preparations were fixed 15 min at room temperature with 4% PFA before X-Gal staining (Relaix et al, 2004) for 3-5 hours or processed for immunofluorescence as previously described (Coletti et al., 2002; Nicolas et al., 2005; Schwarzkopf et al., 2006). Primary antibodies: PW1 (Relaix et al, 1996), β-Gal (Promega), laminin (Sigma). Antibody binding was revealed using species-specific secondary antibodies coupled to Alexa Fluor 488 (Molecular Probes), FITC (DakoCytomation), Cy3 or Cy5 (Jackson Immunoresearch). Nuclei were counterstained with DAPI (Sigma) or nuclear fast red (Sigma).

### 2.2. Results

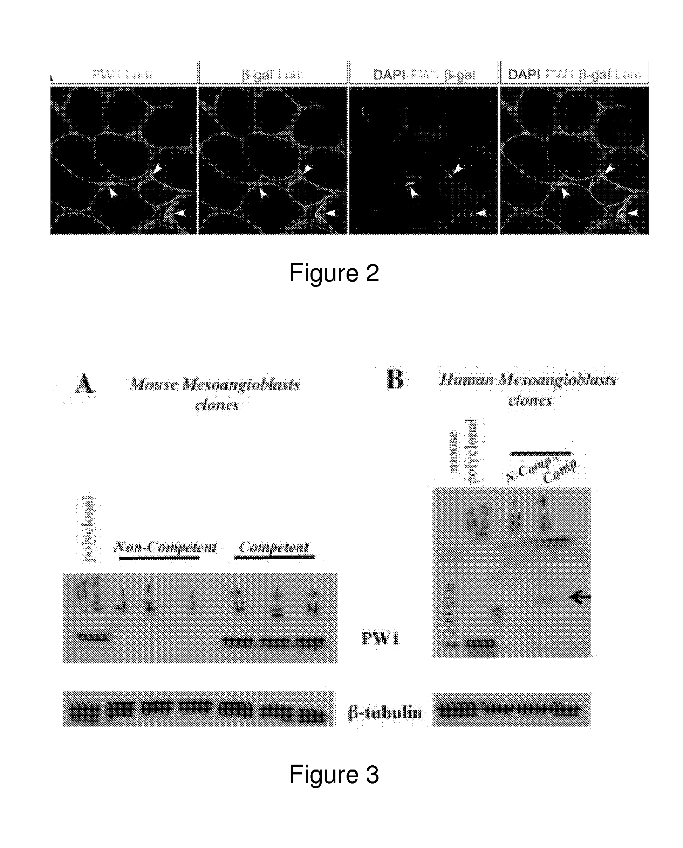

Photomicrographs of representative mouse tissue cross-sections immunostained for PW1 were taken showing the expression of PW1 in tissue specific stem cells (see Figure 2).

In the intestine, immunocytochemical PW1 labeling showed that endogenous PW1 protein expression is restricted to the basal crypt.

Cross sections of the subventricular zone of the brain showed co-expression of PW1 and ß-galactosidase allowing to identify neural stem cells in the brain.

In skin sections immunostained with antibodies against PW1 and keratin 15 a marker of the hair follicle stem cells, PW1 was shown to co-localize with K15, thus defining resident stem cell populations in the skin.

### Example 3: PW1 expression in stem cells cultured in vitro

### 3.1. Materials and methods

The same antibody was used as described in Example 2.1.

### FACs analysis

For fluorescence-activated cell sorting, hematopoietic cells were flushed from the bone marrow of 7 week-old mice (n=10) with PBS containing 1% BSA. Cells were filtrated and treated with 154 mM NH₄Cl, 10 mM KHCO₃ and 0,1 mM EDTA to eliminate red blood cells. Cells were incubated 1 hour at 4°C with 10 ng/ml of the following antibodies: rat anti-mouse hematopoietic lineage flow cocktail-Pacific blue (Lin: CD3, CD45R/B220, CD11b, TER-119, Ly-6G), rat anti-mouse CD34-biotin (Ram34), rat anti-mouse Sca1-PE, rat anti-mouse cKit-APC (all from BD Biosciences). Cell pellets were washed prior to incubation with Streptavidin-PE-Cy7 (BD Biosciences) for 30 min on ice. For detection of nuclear b-galactosidase activity, a fluorescein di-b-D galactopyranoside (FDG) staining kit (Invitrogen) was used according to manufacturer's instructions. Staining analysis was carried out using a FACSAria (Becton Dickinson). ß-galactosidase positive cells were defined as having a signal superior to the signal from the cells isolated from non transgenic mouse.

The PW1 reporter mouse is the mouse generated in Example 1.

### 3.2. Results

Flow cytometric analyses were performed on single cells from the hematopoietic system from the PW1 reporter mouse. Cells were cultured in vitro. Immunofluorescence staining showed co-expression of PW1 and ß-galactosidase.

Immunofluorescence staining was obtained in neurospheres cultured in vitro using PW1 antibody and nestin antibody (classical marker of neural stem cells).

### Example 4: PW1 expression in mouse and human competent stem cells.

### 4.1.Materials and methods

The same antibody was used as described in Example 2.1.

### Western blot

The cells were harvested and lysed in 1 ml of lysis buffer (50 mM Tris-HCl, pH 8.0, 5 mM EDTA, 150 mM NaCl2, 0.5% NP-40, and 1 mM PMSF). The protein extract was subjected to electrophoresis on a 4-12% gradient SDS-polyacrylamide gel. The protein was transferred to a PVDF membrane, probed with anti-PW1 antibody (diluted 1/4000). Immunoblots were developed using horseradish peroxidase (HRP)-conjugated antibodies, followed by detection with enhanced chemiluminescence using an ECL kit (Pierce).

### 4.2. Results

Mesoangioblasts are identified as stem cells, associated with small vessels of the mesoderm in mammals. They are able to proliferate in vitro and to undergo differentiation into various mesoderm cells. These cells have been successfully used in cell transplantation that have yielded a significant rescue in skeletal muscle of dystrophic mice and dogs. The human mesoangioblasts have been identified and isolated. In culture these cells are able to proliferate but only some of the proliferating clones stay competent. Using the anti-PW1 antibody, the inventors have shown that PW1 is expressed only in competent human and mouse mesoangioblasts (see Figures 3A and 3B).

### References

Barker, N., Huch, M., Kujala, P., van de Wetering, M., Snippert, H.J., van Es, J.H., Sato, T., Stange, D.E., Begthel, H., van den Born, M., et al. (2010). Lgr5(+ve) stem cells drive self-renewal in the stomach and build long-lived gastric units in vitro. Cell Stem Cell 6, 25-36.
Blanpain, C., and Fuchs, E. (2006). Epidermal stem cells of the skin. Annu Rev Cell Dev Biol 22, 339-373.
Coletti, D., Yang, E., Marazzi, G., and Sassoon, D. (2002). TNFalpha inhibits skeletal myogenesis through a PW1-dependent pathway by recruitment of caspase pathways. EMBO J 21, 631-642.
Jaks, V., Barker, N., Kasper, M., van Es, J. H., Snippert, H. J., Clevers, H., Toftgard, R. (2008). Lgr5 marks cycling, yet long-lived, hair follicle stem cells. Nat Genet 40, 1291-9.
Kim, J., Ashworth, L., Branscomb, E., and Stubbs, L. (1997). The human homolog of a mouse-imprinted gene, Peg3, maps to a zinc finger gene-rich region of human chromosome 19q13.4. Genome Res 7, 532-540.
Kim, J., Bergmann, A., and Stubbs, L. (2000). Exon sharing of a novel human zinc-finger gene, ZIM2, and paternally expressed gene 3 (PEG3). Genomics 64, 114-118.
Lee, E.C., Yu, D., Martinez de Velasco, J., Tessarollo, L., Swing, D.A., Court, D.L., Jenkins, N.A., and Copeland, N.G. (2001). A highly efficient Escherichia coli-based chromosome engineering system adapted for recombinogenic targeting and subcloning of BAC DNA. Genomics 73, 56-65.
Mitchell, K.J., Pannerec, A., Cadot, B., Parlakian, A., Besson, V., Gomes, E.R., Marazzi, G., and Sassoon, D.A. (2010). Identification and characterization of a non-satellite cell muscle resident progenitor during postnatal development. Nat Cell Biol 12, 257-266. Moore, K.A., and Lemischka, I.R. (2006). Stem cells and their niches. Science 311, 1880-1885.
Morrison, S.J., Shah, N.M., and Anderson, D.J. (1997). Regulatory mechanisms in stem cell biology. Cell 88, 287-298.
Nicolas, N., Marazzi, G., Kelley, K., and Sassoon, D. (2005). Embryonic deregulation of muscle stress signaling pathways leads to altered postnatal stem cell behavior and a failure in postnatal muscle growth. Dev Biol 281, 171-183.
Relaix, F., Rocancourt, D., Mansouri, A., and Buckingham, M. (2004). Divergent functions of murine Pax3 and Pax7 in limb muscle development. Genes Dev 18, 1088-1105.
Relaix, F., Weng, X., Marazzi, G., Yang, E., Copeland, N., Jenkins, N., Spence, S.E., and Sassoon, D. (1996). Pw1, a novel zinc finger gene implicated in the myogenic and neuronal lineages. Dev Biol 177, 383-396.
Schwarzkopf, M., Coletti, D., Sassoon, D., and Marazzi, G. (2006). Muscle cachexia is regulated by a p53-PW1/Peg3-dependent pathway. Genes Dev 20, 3440-3452.
Tajbakhsh, S., Rocancourt, D., Cossu, G., and Buckingham, M. (1997). Redefining the genetic hierarchies controlling skeletal myogenesis: Pax-3 and Myf-5 act upstream of MyoD. Cell 89, 127-138.

## Claims

1. An *in vitro* method for screening a candidate substance for its ability to stimulate adult stem cells, which method comprises the steps consisting of:
a. Providing a tissue or adult stem cells from a subject;
b. Contacting said tissue or cells with the candidate substance;
c. Comparing a binding signal of an anti-PW1 antibody that is detectably labeled, before and after the contacting of said tissue or cell culture with the candidate substance; wherein the ability of the candidate substance to trigger, maintain or enhance the binding signal identifies the substance as being able to stimulate adult stem cells or to maintain competence of adult stem cells.

2. An *in vitro* method for screening a candidate substance for its ability to stimulate adult stem cells, which method comprises the steps consisting of:
a. providing a tissue section or biopsy of a subject who has been administered with the candidate substance;
b. contacting said tissue section or biopsy with an anti-PW1 antibody; wherein the ability of the candidate substance to trigger, maintain or enhance the binding signal of the anti-PW1 antibody that is detectably labeled identifies the substance as being able to stimulate adult stem cells or to maintain competence of adult stem cells.

3. The method of claim 1 or 2, wherein the subject is a non-human animal that has been subjected to a stress, consisting preferably of hypoxia, NO-induced stress, H₂O₂-induced stress, thermal stress, or a chemically induced stress, e.g. with a histone inhibitor.

4. The method according to any of claims 1 to 3, wherein the tissue or stem cells are obtained from a tissue selected from the group consisting of blood, bone marrow, hematopoietic system, skin, hair follicle, muscle, nervous system, heart, intestine, thymus, pancreas, testis, eye, kidney, liver, lung, spleen, tongue, bones, dental pulp, breast, ovaries, uterus, and placenta.

5. The method according to any of claims 1 to 4, wherein the anti-PW1 antibody is detectable by indirect labeling, preferably by an anti-IgG immunoglobulin that is itself detectably labeled.

6. The method according to any of claims 1 to 4, wherein the anti-PW1 antibody is detectable by direct labeling.

7. A method for identifying adult stem cells in a subject, which method comprises
a. obtaining a tissue section or biopsy of a subject;
b. contacting the tissue section of biopsy with anti-PW1 antibody;
c. visualizing the anti-PW1 antibody that is detectably labeled, whereby adult stem cells are identified.

8. The method of claim 7, wherein the tissue section or biopsy is obtained from a tissue selected from the group consisting of blood, bone marrow, hematopoietic system, skin, hair follicle, muscle, nervous system, heart, intestine, thymus, pancreas, testis, eye, kidney, liver, lung, spleen, tongue, bones, dental pulp, breast, ovaries, uterus, and placenta.

9. The method according to any of claims 7, 8, wherein the anti-PW1 antibody is detectable by indirect labeling, preferably by an anti-IgG immunoglobulin that is itself detectably labeled.

10. The method according to any of claims 7, 8, wherein the anti-PW1 antibody is detectable by direct labeling.

11. Use of an anti-PW1 antibody for screening a candidate substance for its ability to stimulate adult stem cells.

12. The use of claim 10, wherein the candidate substance is a chemical drug, a biological compound, or a mixture of natural compounds.

13. Use of an anti-PW1 antibody for monitoring stem cell competence and aging.

14. The use of claim 12, for monitoring stem cell competence during aging.
